# EUROPEAN PATENT SPECIFICATION

(11) **EP 0 729 330 B1**
(45) Date of publication and mention of the grant of the patent: **12.07.2006**
(21) Application number: 95901890.4
(22) Date of filing: 14.11.1994
(51) Int. Cl.: A61F 13/15

(54) **Fibrous and apertured, three-dimensional, macroscopically expanded plastic web**
Faserige, durchlöcherte, dreidimensionale, makroskopische gestreckte Kunststoffbahn
Nappe de plastique expansée de façon macroscopique, tridimensionelle, fibreuse et ajourée

(30) Priority: 18.11.1993 US 154660
(43) Date of publication of application: 04.09.1996
(73) Proprietor: THE PROCTER & GAMBLE COMPANY, Cincinnati, Ohio 45202 (US)
(72) Inventor: COOPER, John, Thomas, West Chester, OH 45069 (US); LITCHHOLT, John, Joseph, Harrison, OH 45030 (US); GRAY, Brian, Francis, Burlington, Ontario L7M 3E9 (CA); SCHETTLER, Michael, John, Barrie, Ontario L4N 4W9 (CA); ASHTON, Gregory, Markham, Ontario L3P 6T8 (CA)
(74) Representative: Veronese, Pancrazio
(86) International application number: PCT/US1994/013097
(87) International publication number: WO 1995/013773

(56) References cited:
- EP-A- 0 409 535
- WO-A-93/09741
- US-A- 3 965 906
- US-A- 4 342 314
- US-A- 5 171 238

## Description

### FIELD OF THE INVENTION

The present invention relates to absorbent articles such as sanitary napkins, pantiliners, disposal diapers, incontinent articles, and the like, and more particularly, the present invention relates to absorbent articles having a fibrous and apertured, three-dimensional, macroscopically expanded, plastic topsheet.

### BACKGROUND OF THE INVENTION

AD manner and variety of absorbent articles configured for the absorption of bodily fluids are, of course, well known. Current types of absorbent articles include sanitary napkins, pantiliners, disposable diapers, and incontinent articles.

Macroscopically expanded, three-dimensional, apertured, films have been used as topsheets on such absorbent articles. As used herein, the term ''maeroscopically expanded", when used to describe three-dimensional plastic webs, ribbons and films, refers to webs, ribbons and films, which have been caused to conform to the surface of a three-dimensional forming structure so that both surface thereof exhibit thethree-dimensional pattern of the forming structure, the pattern being readily visible to the naked eye when the perpendicular distance between the viewer's eye and the plane of the web is about 305mm (12 inches).

One macroscopically expanded, three-dimensional, apertured plastic web which is particularly well suited to transferring fluid deposited on one surface thereof to its opposite surface and thereafter isolating the transferred fluid from the wearer's skin is disclosed in commonly assigned U.S. Pat. No. 3, 929,135 issued to Thompson on Dec. 30, 1975. Thompson describes a macroscopically expanded, tbree-dimensional, topsheet comprised of liquid impermeable material, but provided with a pattern of tapered capillaries, the capillaries have a base opening in the plane of the topsheet contacting the wearer's body and an apex opening remote from the base opening of the topsheet, the apex opening being in intimate contact with the absorbent pad utilised in the disposable absorbent article. The Thompson topsheet allows for the free transfer of fluid from the wearer's body into the absorbent element of the device while inhibiting the reverse flow of these fluids. This provides a relatively much dryer surface in contact with the user than had previously been obtainable.

Another macroscopically expanded, three-dimensional, apertured plastic web well suited for use as a topsheet on absorbent articles such as sanitary napkins is disclosed in commonly assigned U.S. Pat. No. 4,342,314 issued to Radel et al. on Aug. 3, 1982. The macroscopically expanded, three-dimensional plastic web disclosed in the Radel et al. patent exhibits a fiberlike appearance and tactile impression which has been favorably received by consumers when used as a wearer contacting surface.

While macroscopically expanded, three-dimensional, apertured plastic webs have achieved widespread commercial success when employed as topsheets on absorbent articles, some users are very reluctant to place a topsheet which they readily perceive as plastic in contact with their skin.

European Patent Specification No. EP-A-0 409 535 discloses a non-woven fabric comprising a base cloth layer made of thermoplastic fiber-like filaments and having a number of holes. Cylindrical film projections project from the peripheral edge of each hole which projections are also made of thermoplastic fiber-like filaments. The height of the projection is at least twice the thickness of the base cloth layer. In a preferred embodiment, filaments of the thermoplastic resin material are blown from a die against the resin film to form an addition base layer on the base cloth layer and cylindrical projections within each of the cylindrical film projections.

International Patent Specification No. WO-A-9309741 discloses an absorbent article having a topsheet comprising of a non-woven material and apertured thermoplastic film.

Accordingly, it is therefore an object of the present invention to provide a topsheet for an absorbent article having fluid handling characteristics comparable to those found in macroscopically expanded, three-dimensional, apertured plastic topsheets and an improved softer, less plastic-like feel.

### SUMMARY OF THE INVENTION

The present invention relates to absorbent articles such as diapers, incontinent articles, sanitary napkins, and the like. More particularly, this invention relates to absorbent articles having a composite fibrous and macroscopically expanded, three dimensional, apertured plastic film topsheet. The resilient, three-dimensional, macroscopically expanded, fluid pervious plastic web has a first surface and a second surface located in planes remote from one another. A plurality of capillaries extend from the first surface to the second surface of the plastic web. The capillaries are defined by a plurality of sidewall portions interconnected to one another intermediate the first and second surfaces and terminate in the second surface. A plurality of fibers are secured to the first surface of the fluid pervious plastic web. The fibers extend into the capillaries of the fluid pervious plastic web and are secured to the sidewall portions.

The present invention also provides a process for forming a fibrous and apertured, three dimensional, macroscopically expanded, plastic web. A polymeric film is extruded onto a forming structure. The forming structure exhibits a macroscopic, three-dimensional, cross-section defined by a multiplicity of macroscopic apertures which place the opposed surface of the forming structure in fluid communication with one another. A plurality of fibers are deposited on the film while the film is supported by the forming structure to form a composite web. A pneumatic pressure differential is applied to the composite web of film and fibers to cause the composite web to be urged into substantial conformance with the macroscopic, three-dimensional, cross-section of the forming structure forming a three-dimensional, macroscopically expanded, web having a plurality of capillaries extending from the first surface to said second surface. The capillaries are defined by a plurality of sidewall portions interconnected to one another intermediate the first and second surfaces and which terminate in the second surface. The pneumatic pressure causes the fibers to be pulled against the first surface of the formed web and into the capillaries of the formed web. The fibers are secured to the first surface and the sidewall portions of said formed web forming a fibrous and apertured, three-dimensional, macroscopically expanded web.

### BRIEF DESCRIPTION OF THE DRAWINGS

While the specification concludes with claims particularly pointing out and distinctly claiming the present invention, it is believed that the present invention will be better understood from the following description in conjunction with the accompanying drawings in which:
Fig. 1 is a simplified perspective view of an absorbent article of the present invention;
Fig. 2 is a cross-sectional view taken along section line 2-2 of Fig. 1;
Fig. 3 is a cross-sectional view taken along section line 3-3 of Fig. 1;
Fig. 4 is an enlarged, partially segmented, perspective illustration of a prior art plastic web of the type generally enclosed in commonly assigned U.S. Pat. No. 4,342,314;
Fig. 5 is an enlarged, partially segmented, perspective illustration of a preferred embodiment of the fibrous and apertured, plastic web of the present invention;
Fig. 6 is an enlarged, cross-sectional view of a fiber-like element of the fibrous and apertured, plastic web of the present invention taken along section line 6-6 of Fig. 5;
Fig. 7 is a simplified schematic representation of a process for making the fibrous and apertured, plastic web of the present invention;
Fig. 8 is a greatly enlarged fragmentary view of the forming structure utilized to support the fibrous and apertured, plastic web in accordance with the process illustrated in Fig. 7;
Fig. 9 is a simplified schematic representation of an alternative process for making the fibrous and apertured, plastic web of the present invention; and
Fig. 10 is a simplified schematic representation of an alternative process for making the fibrous and apertured, plastic web of the present invention.

### DETAILED DESCRIPTION OF THE PRESENT INVENTION

The term "absorbent article", as used herein, refers to articles which absorb and contain body exudates. More specifically, the term refers to articles which are placed against or in proximity to the body of the wearer to absorb and contain the various exudates discharged from the body. The term "absorbent article" is intended to include diapers; incontinent articles, sanitary napkins, pantiliners, and other articles used to absorb body exudates. The term "disposable "is used herein to describe absorbent articles which are not intended to be laundered or otherwise restored or reused as an absorbent article, i.e., they are intended to be discard after a single use, and, preferably to be recycled, composted or otherwise disposed of in an environmentally compatible manner.

The term "diaper" refers to a garment generally worn by infants and incontinent persons which is worn about the lower torso of the wearer. Suitable diapers that can be provided with the topsheet described herein are disclosed in U.S. Pat. No. Re. 26,152, issued to Duncan, et al. on Jan. 31, 1967; U.S. Pat. No. 3,860,003 issued to Buell on Jan. 14, 1975; U.S. Pat. No. 4,610,678 issued to Weisman, et al. on Sept. 9, 1986, U.S. Pat. No. 4,673,402 issued to Weisman, et al. on June 16, 1987; U.S. Pat. No. 4,695,278 issued to Lawson on Sept. 22, 1987; U. S. Pat. No. 4,704,115 issued to Buell on Nov. 3, 1987; U.S. Pat. No. 4,834,735 issued to Alemany et al. on May 30, 1989; U. S. Pat. No. 4,888,231 issued to Angstadt on Dec. 19, 1989; and U. S. Pat. No. 4, 909,803 issued to Aziz, et al. on March 20, 1990.

The term "incontinent article" refers to pads, undergarments (pads held in place by a suspension system of some type, such as a belt, or the like), inserts for absorbent articles, capacity boosters for absorbent articles, briefs, bed pads, and the like, regardless of whether they are worn by adults or other incontinent persons. Suitable incontinent articles that can be provided with the topsheet described herein are disclosed in U.S. Pat. No. 4,253,461 issued to Strickland, et al. on March 3, 1981; U.S. Pat. Nos. 4,597,760 and 4,597,761 issued to Buell; the above-mentioned U.S. Pat No. 4,704,115; U.S. Pat. No. 4,909,802 issued to Ahr, et al.; and U.S. Pat. No. 4,964,860 issued to Gipson, et al. on Oct. 23, 1990; each of which is incorporated herein by reference.

The term "sanitary napkin" refers to an article which is worn by females adjacent to the pudendal region that is intended to absorb and contain various exudates which are discharged from the body (e.g., blood, menses and urine). Suitable sanitary napkins that can be provided with the topsheet described herein are disclosed in U.S. Pat. No. 4,285,343, issued to McNair on Aug. 25, 1981; U.S Pat. Nos. 4,589, 876 and 4,687,478 issued to Van Tilburg on May 20, 1986 and Aug. 18, 1987 respectively; U.S Pat. Nos. 4,917,697 and 5,007,906 issued to Osborn, et al, on April 17, 1990 and April 16, 1991, respectively; and U.S. Pat. Nos. 4,950,264 and 5,009,653 issued to Osborn on Aug. 21, 1990 and April 23, 1991.

The term "pantiliner" refers to absorbent articles that are less bulky than sanitary napkins which are generally worn by women between their menstrual periods. Suitable pantiliners that can be provided with the topsheet described herein are disclosed in U.S. Pat. No. 4,738,676 issued to Osborn on April 19, 1988.

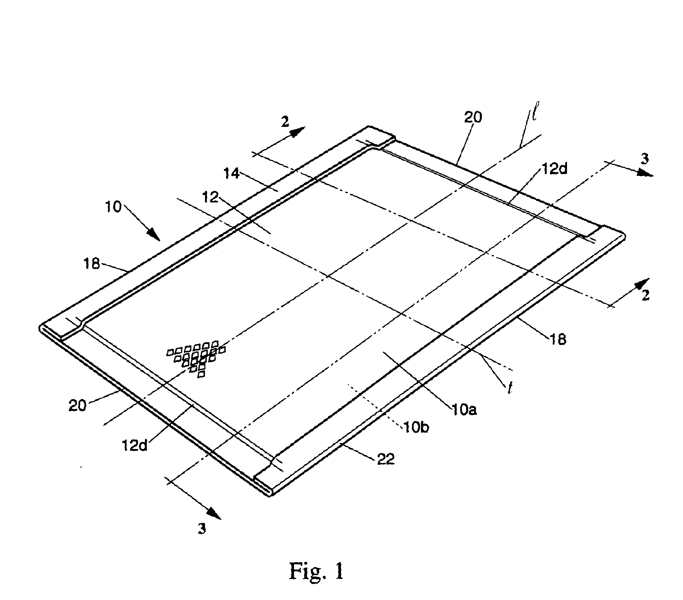

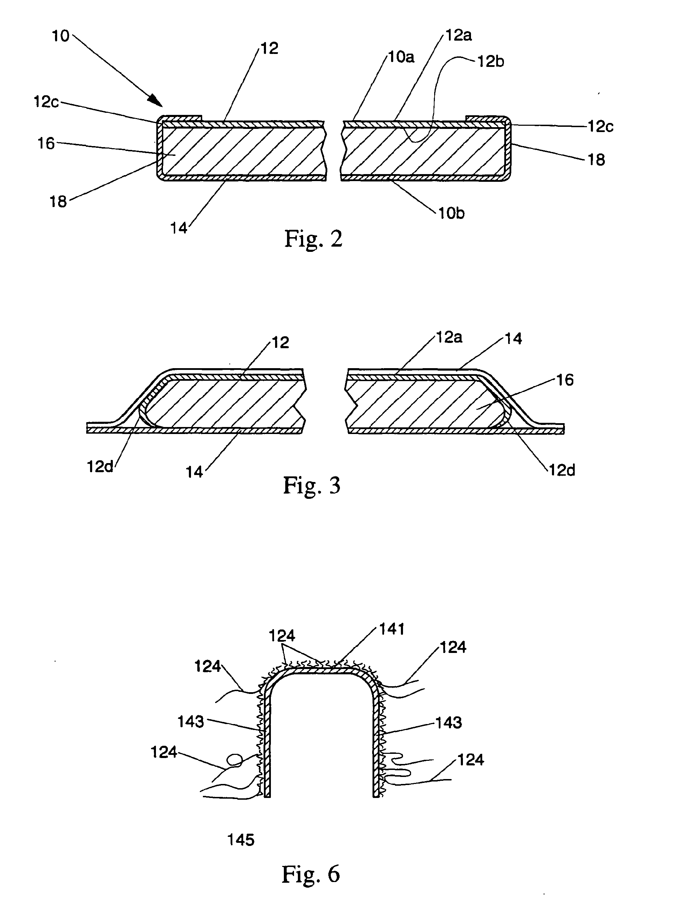

Fig. 1 shows a simplified absorbent article 10 that could represent a diaper prior to its being placed on a wearer. It should be understood, however, that the present invention is not limited to the particular type or configuration of absorbent article shown in the drawings. As shown in Figs. 2 and 3, such an absorbent article 10 basically comprises a topsheet 12, a backsheet 14, and an absorbent core 16.

The absorbent article 10 has two surfaces, a body-contacting surface (or "body surface") 10a and a garment surface 10b. The body surface 10a is intended to be worn adjacent to the body of the wearer. The garment surface 10b of the absorbent article 10 (shown in Fig. 2) is on the opposite side and is intended to be placed adjacent to the wearer's undergarments or clothing when the absorbent article 10 is worn.

The absorbent article 10 has two centerlines, a longitudinal centerline 1 and a transverse centerline t. The term "longitudinal", as used herein, refers to a line, axis or direction in the plane of the absorbent article 10 that is generally aligned with (e.g:, approximately parallel to) a vertical plane which bisects a standing wearer into left and right body halves when the absorbent article 10 is worn. The terms "transverse" or "lateral" as used herein, are interchangeable, and refer to a line, axis or direction which lies within the plane of the absorbent article 10 that is general perpendicular to the longitudinal direction. Fig. 1 shows that the absorbent article 10 has two spaced apart longitudinal edges 18 and two spaced apart transverse or end edges (or "ends") 20, which together form the periphery 22 of the absorbent article 10.

The topsheet 12 is compliant, soft-feeling and non-irritating to the wearer's skin. Further, topsheet 12 is liquid permeable, permitting liquids to readily penetrate through its thickness. The topsheet 12 has a body-facing side or face 12a and a garment-facing side or face 12b, two longitudinal or side edges 12c and two end edges 12d. (A similar numbering system will be used for the other components of the diaper 10. That is, the side of the component facing the wearer's body will be designated by the number of the component and a reference letter "a", the side facing the wearer's undergarments by the number of the component and the letter "b", and the side and end edges by the number of the component and the reference letters "c" and "d" respectively.)

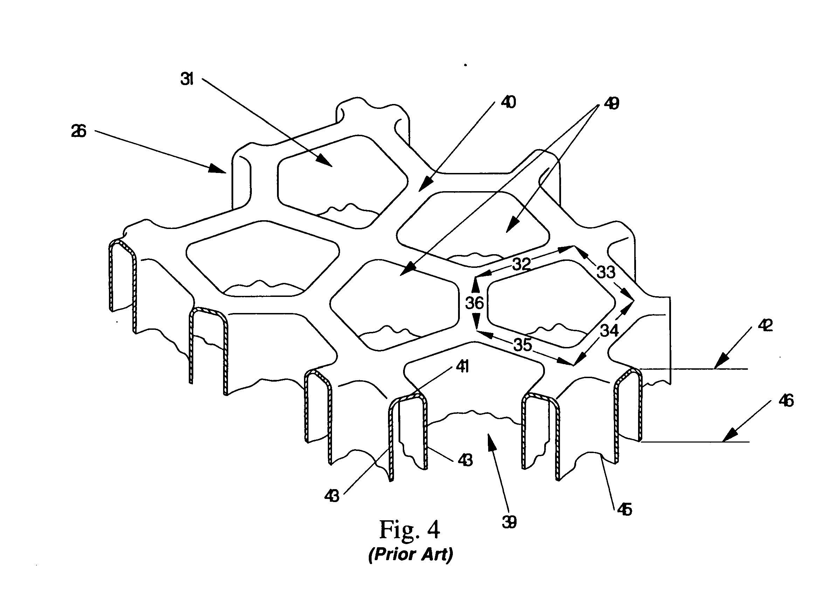

Fig. 4 is an enlarged, partially segmented, perspective illustration of a prior art macroscopically expanded, three-dimensional, fiber-like, apertured, plastic film 26 which has been found highly suitable for use as a topsheet in disposal absorbent articles. Prior art plastic film 26 is generally in accordance with the teachings of commonly assigned U.S. Pat. No. 4,342,314 issued to Radel, et al. on Aug. 3, 1982. The plastic film 26 exhibits a multiplicity of apertures, e.g., apertures 31, which are formed by a multiplicity of intersecting fiber-like elements, e.g., elements 32, 33, 34, 35 and 36 interconnected to one another in the first surface 40 of the web. Each fiber-like element comprises a base portion, e.g., base portion 41 located in plane 42. Each base portion has a sidewall portion, e.g., sidewall portions 43, attached to each edge thereof. The sidewall portions extend generally in the direction of a second surface 45 of the web. The intersecting sidewall portions of the fiber-like elements are interconnected to one another intermediate the first and second surfaces of the web, and terminate substantially concurrently with one another in the plane 46 of the second surface to form apertures 39 in the second surface 45 of the web. The capillaries 49 formed by the interconnected sidewall portions allows for free transfer of fluid from the first surface of the web directly to the second surface of the web without lateral transmission of the fluid between the adjacent capillaries.

The apertured plastic film 26 is manufactured from a liquid impervious, preferably thermoplastic material. The thermoplastic material for use in the manufacture of the apertured plastic film 26 is selected from a group generally consisting of polyethylene, polypropylene, polyvinyl chloride, starch base resins, polyvinyl alcohol, polyurethanes, polycaprolactone cellulose esters and blends thereof.

Examples of other macroscopically expanded, three-dimensional, apertured, plastic webs are disclosed in U.S. Pat. No. 3,939,135 issued to Thompson on Dec. 30, 1975; U.S. Pat. No. 4,324,426, issued to Mullane, et al. on April 13, 1982; and U.S. Pat. No. 4,463,045, issued to Ahr, et al on July 31, 1984.

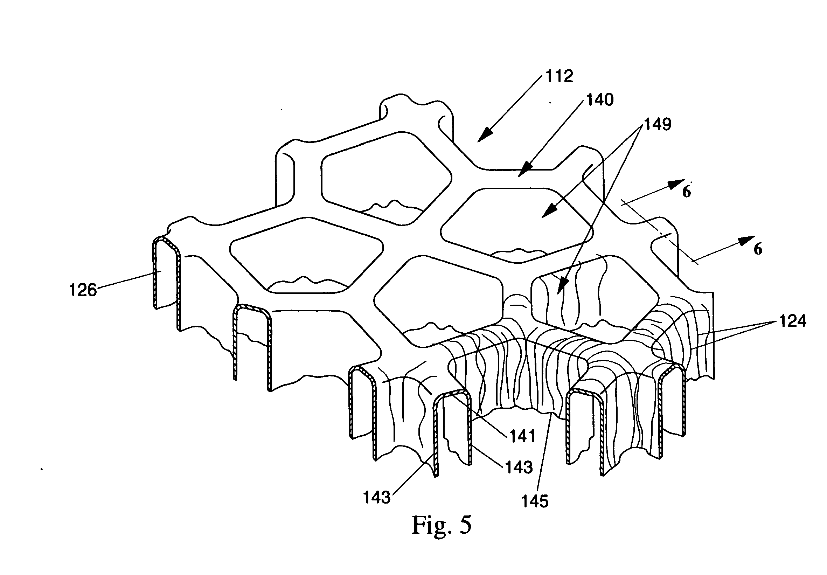

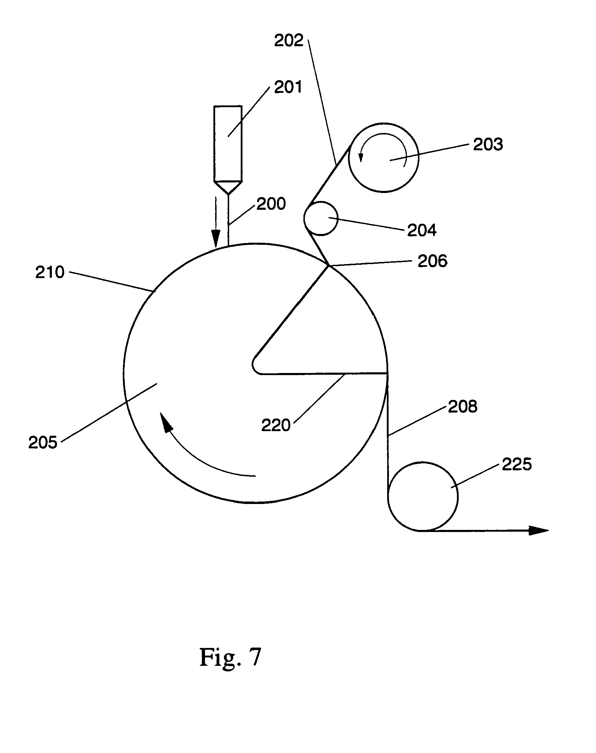

Fig. 5 is an enlarged, partially segmented, perspective illustration of a particularly preferred embodiment of a fibrous and apertured, plastic web 112. Fibrous and apertured plastic web 112 is particularly well suited for use as a topsheet, e.g., topsheet 12 illustrated in Fig. 1, on an absorbent article, such as a diaper or sanitary napkin. Web 112 comprises a plurality of fibers 124 secured to a macroscopically expanded, three-dimensional, apertured, plastic web 126. Apertured plastic web 126 is generally in accordance with the teachings of commonly assigned U.S. Pat. No. 4,342,314 issued to Radel et al. Referring now to Fig. 6, the fibers 124 are secured to the apertured plastic web 126 along the first surface 140 and extend substantially into the capillaries 149 where a substantial portion of the fibers 124 are secured to the sidewall portions 143 of the apertured plastic film 126. Despite the presence of fibers 124 the appearance of web 112, is generally similar to that of prior art web 26 illustrated in Fig. 4, that is, the pentagonal shape of capillaries 49 of web 26 is similar to the pentagonal shape of capillaries 149 of web 112.

By securing the fibers 124 to both the first surface 140 and the sidewall portions 143 of the apertured plastic web 126, a substantial portion of the fibers 124 do not span the capillaries 149, where the fibers would possibly hinder the transfer of bodily fluids from the first surface 140 to the second surface 145 of the web. By leaving the capillaries substantially open and free of fibers, the fibrous and apertured, composite web 112 is able to handle heavy or viscous fluids such as runny BM, when the composite web is employed as a topsheet on a disposable diaper. Some fibers 124 may span across the capillaries 149, but they will not substantially hinder the transmission of bodily fluids from the first surface 140 to the second surface 145 of the web.

Due to the securement of the fibers 124 to the first surface 140 and the sidewall portions 143 of the apertured plastic web 126, the composite web 112 has an open area approaching that of apertured plastic web 126. Therefore, fluid deposited on the first surface 140 is transmitted from the first or wearer contacting surface to the second or pad contacting surface 145 of the composite web 112.

The addition of the fibers 124 to the first or wearer contacting surface of the apertured plastic web 126 provides a textured surface which when utilized as a topsheet on an absorbent article is in contact with the wearer's skin. The tactile perception of the web 112 is similar to that of a fibrous nonwoven web. The addition of the fibers to the wearer contacting surface of the apertured plastic film 126 reduces the plastic feel associated with such films which some users resist placing in contact with their skin.

The addition of the fibers 124 to the wearer contacting surface of the apertured plastic film 126 also provides the web 112 with a visual distinction from the plastic web 126. That is, the fibers provide a roughness or an irregularity such that light incident upon the visible surface of the web 15 substantially diffused into a multiplicity of directions rather than being speculary reflected, thereby providing a non-glossy visible surface which reduces the wearer's perception that the web is comprised of plastic.

In one preferred embodiment, the fibers 124 may be in the form of a nonwoven fabric or web. The nonwoven fabric may be manufactured from a wide range of material such as natural fibers (e.g., wood, or cotton fiber), synthetic fibers (e.g., polyester, polypropylene) or a combination thereof. The nonwoven fabric is preferably made from fibers selected from a group consisting of polypropylene, polyester, polyethylene polyvinyl alcohol, starch base resins, polyurethanes, cellulose and cellulose esters.

There are a number of manufacturing techniques which may be utilized to manufacture the nonwoven fabric. For example, nonwoven fabric may be resin-bonded, needle punched, spunbonded, carded, the latter including, thermally bonded, air-thru bonded, and spunlaced fabrics. A preferred nonwoven fabric is a thermally bonded polypropylene fabric.

The nonwoven fabric preferably has a relatively low basis weight ranging from about 1.0g/sq.m. to about 22 g/sq.m. For one embodiment, the nonwoven fabric has a basis weight range of from about 15 to about 17 grams per square yard (about 18 to about 20 g/sq.m.) and a caliper of from about 3 to about 5 mils (about 0.76 to about 0.12 mm.) when measured under a load of about 200 pascals. Such a nonwoven fabric is further characterized by a maximum wet or dry tensile strength of less than about 600 grams per centimeter in the longitudinal or machine direction and less than about 150 grams per centimeter in the cross machine direction.

In another preferred embodiment, the nonwoven fabric has a lighter weight of from about 8 g/sq.m. to about 10 g/sq.m. Such lighter nonwoven fabrics are highly preferred. They are preferred because they can be used (with a lightweight film) to form a composite fibrous and apertured, plastic web 112 that is sufficiently thin and lightweight that it behaves as a single sheet of material. This provides the advantages of being more flexible and requiring the use of smaller amounts of raw materials.

In another preferred embodiment, the fibers 124 may be meltblown onto the plastic web 126. Preferably, the meltblown fibers 124 are deposited on the plastic web in a range of basis weights up to about 22 g/sq.m. and most preferably in a range of basis weights from about 2.0 g/sq.m. to about 5.0 g/sq.m. The meltblown fibers may be selected from a wide range of materials selected from the group consisting of polypropylene, polyester, polyethylene polyvinyl alcohol, starch base resins, polyurethanes, cellulose and cellulose esters. A detailed description of this particular method of securing the fibers 124 to the plastic web 126 is described in greater detail below.

A suitable process of preparing the fibrous and apertured, plastic web 112 of the present invention is shown in Fig. 7. In the embodiment shown in Fig. 7, a web of molten resin 200 is extruded from a conventional extruder 201 onto the surface of a forming drum 205 about which a forming structure 210 rotates. A web of nonwoven fabric 202 is fed from a supply roll 203 over nip roll 204 onto the web of molten resin 200 located on forming structure 210. The web of nonwoven fabric 202 adheres to the semi-molten resin forming a composite web 206. The forming drum 205 preferably includes an internally located vacuum chamber 220 which is preferably stationary relative to the moving forming structure 210. As the composite web 206 of semi-molten resin 200 and nonwoven fabric 202 passes across vacuum chamber 220 a pneumatic pressure is applied to the composite web to cause the composite web to be urged into substantial conformance with the forming structure 210.

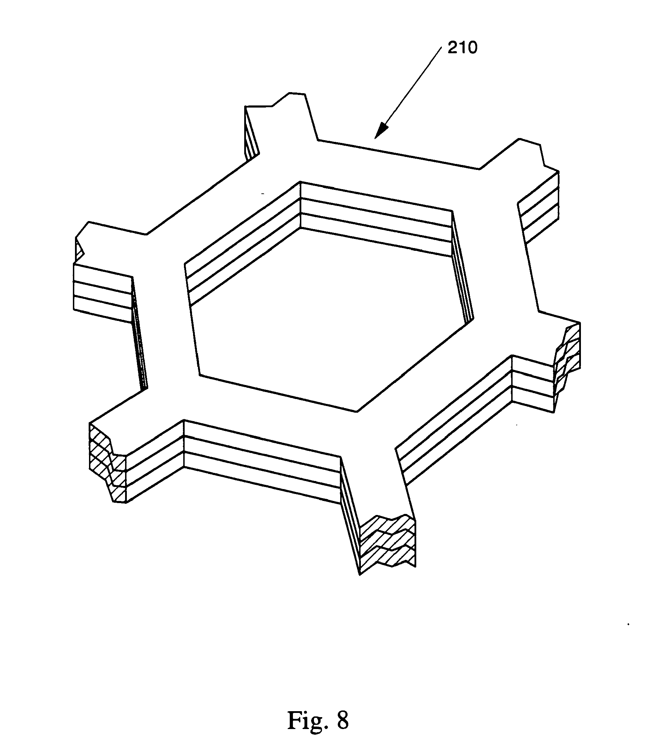

The macroscopic cross-section of forming structure 210 is visible in the greatly enlarged fragmentary perspective illustration of Fig. 8. Methods of constructing a suitable three-dimensional tubular forming member similar to that illustrated in Fig. 8 are disclosed in commonly assigned U.S. Pat. No. 4,508,256 issued to Radel et al. on Apr. 2, 1985 and in commonly assigned U.S. Pat. No. 4,509,908 issued to Mullane, Jr. on Apr. 9, 1985.

As the composite web 206 of semi-molten resin 200 and nonwoven fabric 202 conforms with the macroscopic cross-section of forming structure 210 a fibrous and three-dimensional, macroscopically expanded, apertured web is created similar to web 112 illustrated in Fig. 5. The web has a first surface and a second surface located in planes remote from one another. The formed web includes a plurality of capillaries which extend from the first surface to the second surface. The capillaries are defined by a plurality of sidewall portions interconnected to one another intermediate the first and second surfaces and which terminate in the second surface of the web.

The pressure of vacuum chamber 220 causes the nonwoven fabric to be pulled against the first surface of the plastic web and down into the capillaries of the plastic web. The pressure applied by the vacuum chamber 220 also helps to bond or secure the nonwoven fabric to the base portions and the sidewall portions of the first surface of the plastic web.

The composite web 208 is preferably treated with an effective amount of surface active agent or surfactant. The surfactant provides the nonwoven fabric's surface with greater polararizability than it would have without the surfactant being added. Higher surface polarity yields higher wet ability. Suitable surfactants include a product known commerciatly ATNER645 manufactured by ICIS Specialty Ch. The nonwoven fabric may be treated with a surfactant prior to or during the time it is manufactured. For example, it may be treated after it is unwound from feedroll 203. The surfactant may be applied by any know technique, such as spraying, padding, or by the use of transferals. The surfactant can alternatively (or additionally) be incorporated into the nonwoven fabric such as between or within the fibers of the nonwoven fabric. Preferably, the nonwoven is treated with a surfactant prior to the time it is supplied in the present process.

After the nonwoven fabric and apertured plastic film have been have been formed into composite web 208, composite web 208 is removed from forming drum 210 by way of nip roll 225. The composite web 208 may be fed to a rewind station for temporary storage or may be utilized without further processing as a topsheet in an absorbent article.

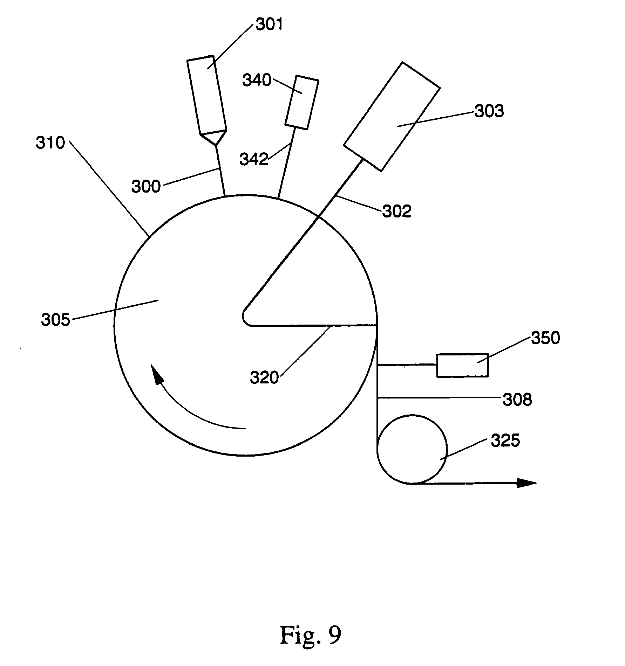

Another suitable process for preparing the topsheet 112 is shown in Fig. 9. In the embodiment shown in Fig. 9, a web of molten resin 300 is extruded from a conventional extruder 301 onto the surface of a forming drum 305 about which a forming structure 310 rotates. A stream of meltblown fibers 302 is blown from die 303 onto the web of molten resin 300 which is supported by forming structure 310. The forming drum 305 preferably includes an internally located vacuum chamber 320 which is preferably stationary relative to the moving forming structure 310. As the molten resin 300 and the meltblown fibers 302 pass across vacuum chamber 320 a pneumatic pressure is applied to the molten resin and the meltblown fibers to cause the resin along with the fibers to be urged into substantial conformance with the forming structure 310. The forming structure 310 is similar to forming structure 110 illustrated in Fig. 7.

In the embodiment of Fig. 9, a non-water soluble adhesive 342 may be added from applicator 340 onto molten resin 300 prior to the addition of meltblown fibers 302. The adhesive 342 helps ensure the attachment of meltblown fibers 302 to molten resin 300.

To create a topsheet similar to that illustrated in Fig. 5, where the nonwoven layer does not span across or block the capillaries of the apertured plastic film, the nonwoven or fibrous matter must be able to conform with the general shape of the macroscopically expanded, three-dimensional forming structure. To permit the nonwoven or fibrous matter to conform with the shape of the forming structure it must not be so dense or tightly bonded such that the individual fibers are not permitted to be pulled down into the capillaries of the apertured plastic film where they are secured with the sidewall portions of the apertured plastic web.

In another embodiment, the stream of meltblown fibers may be applied to a molten resin after the resin has been formed into a macroscopically expanded, three-dimensional, apertured plastic web, similar to that illustrated in Fig. 4. This embodiment is illustrated in Fig. 9, when die 350 is positioned downstream of the vacuum forming operation. The fibers must be blown from a die with such a force that they are able to extend in the capillaries of the macroscopically expanded, three-dimensional, apertured, plastic web. By varying the velocity of the meltblown fibers, the effective momentum and it turn the depth of in penetration of the meltblown fibers into the capillaries of the plastic web can be varied. The fibers must be heated to such a temperature or the apertured, plastic web must be heated such that the meltblown fibers will adhere to the apertured, plastic web.

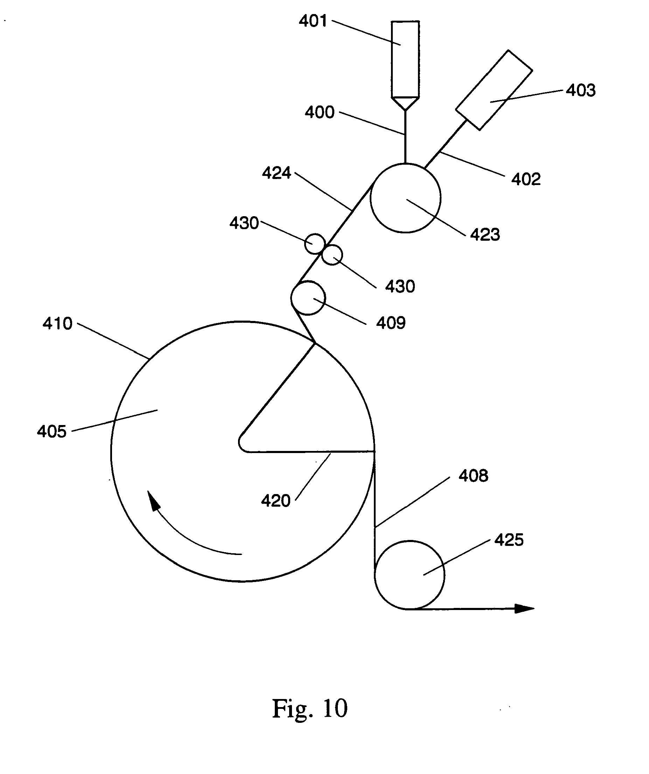

Another process for preparing the fibrous and apertured, plastic web of the present invention is shown in Fig. 10. A stream of meltblown fibers 402 is blown from die 403 onto the surface of drum 423. A web of molten resin 400 is extruded from a conventional extruder 401 onto meltblown fibers 402 to create a composite web 424. Composite web 424 is fed over nip roll 409 onto the surface of a forming drum 405 about which a forming structure 410 rotates such that the molten resin side of composite web 424 is in contact with forming structure 410. As the composite web 424 passes across internally located vacuum chamber 420 a pneumatic pressure is applied to composite web 424 to cause the composite web to be urged into substantial conformance with forming structure 410. After forming, web 408 is removed from forming drum 405 by way of nip roll 425.

In the process illustrated in Fig. 10, a set of calendaring rolls 430 may be added to help ensure that the composite web remains in tact. In the embodiment illustrated in Fig. 10, the calendaring rolls 430 are added prior to the composite web reaching the forming drum 405. Calendaring rolls may also be added after the formed web 408 is removed from forming drum 405.

The absorbent core 16 is positioned between the topsheet 12 and the backsheet 14. The absorbent core 16 may be any absorbent means which is capable of absorbing or retaining liquids (e.g., menses and/or urine). As shown in Figs. 1-3, the absorbent core 16 has a body surface, a garment surface, side edges and end edges. The absorbent core 16 may be manufactured in a wide variety of sizes and shapes (e.g., rectangular, oval, hourglass, dog bone, asymmetric, etc.) and from a wide variety of liquid-absorbent materials commonly used in absorbent articles such as comminuted wood pulp which is generally referred to as airfelt. Examples of other suitable materials include creped cellulose wadding, cross-linked cellulose fibers, absorbent foams, absorbent sponges, synthetic staple fibers, polymeric fibers, hydrogel-forming polymer gelling agents, peat moss, combinations of the foregoing, or any equivalent material or combinations of materials. The configuration and construction of the absorbent core may also be varied (e.g., the absorbent core may have varying caliber zones (e.g., profiled so as to be thicker in the center) hydrophilic gradients, super absorbent gradients, or lower density and lower average basis weight acquisition zones); or may comprise one or more layers or structures. The total absorbent capacity of the absorbent core should, however, be compatible with the desired loading and the intended use of the absorbent article. Further, the size and absorbent capacity of the absorbent core may be varied to accommodate different uses such as incontinence pads, pantiliners, regular sanitary napkins, or overnight sanitary napkins.

Suitable cross-linked cellulose fibers are described in U.S. Pat. No. 4,888,093, issued to Cook, et al. on Dec. 19, 1989; U.S. Pat. No. 4,822,543, issued to Dean, et al. on April 18, 1989; U.S. Pat. No. 4,889,595, issued to Schoggen, et al. on Dec. 26, 1989; U.S. Pat. No. 4,889,596, issued to Schoggen, et al. on Dec. 26, 1989; U.S. Pat. No. 4,898,642 issued to Moore, et al. on Feb. 6, 1990; U.S. Pat. No. 4,935,022, issued to Lash, et al. on June 19, 1990.

The characteristics of the absorbent core 16 for particular types of absorbent articles are described in greater detail in the patents and documents incorporated by reference herein, and the patents and other documents incorporated by reference in those documents, the disclosures of which are all incorporated by reference herein. Other suitable absorbent core arrangements are described in U.S. Pat. Nos. 4,988,344 and 4,988,345, and European Patent Application Publication No. 0 198 683, published Oct. 22, 1986 in the name of Duenk, et al. The absorbent article could also include any additional layers or other components such as are described in the patents incorporated by reference. For example, the absorbent article 10 may comprise an acquisition layer or patch of cross-linked cellulose fibers positioned between the topsheet 12 and the absorbent core 16.

The backsheet 14 and the topsheet 12 are positioned adjacent to the garment surface and the body surface, respectively, of the absorbent core 16 and are preferably joined thereto and to each other by attachment means (not shown) such as those well known in the art. For example, the backsheet 14 and/or the topsheet 12 may be secured to the absorbent core 16 by a uniform continuous layer of adhesive, a patterned layer of adhesive, or an array of separate lines, spirals, spots of adhesive, or meltblown adhesives. Adhesives which have been found to be satisfactory are manufacture by HB Fuller Company of St. Paul, Minnesota under the designation HL-1258. The attachment means will preferably comprise an open pattern network of filaments of adhesives as is disclosed in U.S. Pat. No. 4,573,986 issued to Minetola et al. on March 4, 1986, and which is incorporated herein by reference An exemplary attachment means of an open pattern network of filaments comprises several lines of adhesive filaments swirled into a spiral pattern such as illustrated by the apparatus and method shown in U.S. Pat. No. 3,911,173 issued to Sprague, Jr. on October 7, 1975; U.S. Pat. No. 4,785,996 issued to Zieker et al. on November 22, 1978; and U.S. Pat. No. 4,842,666 issued to Werenicz on June 27, 1989.

Alternatively, the attachment means may comprise heat bonds, pressure bonds, ultrasonic bonds, dynamic mechanical bonds, or any other suitable attachment means or combinations of these attachment means as are known in the art.

The backsheet 14 is impervious to liquids and is preferably manufactured from a thin plastic film, although other flexible liquid impervious materials may also be used. The backsheet 14 prevents liquid contained in absorbent core 16 from wetting articles which contact the absorbent article 10. Polyethylene films having a thickness of from about 0.001 to about 0.002 inches (0.0025 to 0.0051 cm.) have been used for the backsheet 14 with satisfactory results. As used herein, the term "flexible" refers to materials which are compliant and which will readily conform to the general shape and contours of the human body.

The backsheet 14 is superimposed on the garment-facing side 16b of absorbent core 16 and preferably extends beyond the edges thereof. The topsheet 12 is superimposed over the body-facing side 16a of the absorbent core 16, and may also extend beyond the edges of the core 16. The absorbent core 16 is, therefore, positioned between the topsheet 12 and the backsheet 14. The topsheet 12 and backsheet 14 are joined to each other such as around their peripheries. The topsheet 12 and backsheet 14 can be joined in any suitable manner such as by the use of adhesives, crimping, heat-sealing, or ultrasonic bonding.

## Claims

1. A resilient, three-dimensional, macroscopically expanded, fluid pervious plastic web (112) having first (140) and second (145) surfaces located in planes remote from one another, said web (112) having a plurality of capillaries (149) extending from said first surface (140) to said second surface (145), said capillaries (149) being defined by a plurality of sidewall portions (143) interconnected to one another intermediate said first (140) and second (145) surfaces, said sidewall portions (143) terminating in said second surface (145);
a plurality of fibers (124) secured to said first surface (140) of said web (112), **characterised by** said fibers (124) extending into the capillaries (145) and a substantial portion of said fibers (124) being secured to said sidewall portions (143).

2. A fluid pervious web (112) as claimed in claim 1, wherein said fibers (124) are in a nonwoven fabric.

3. A fluid pervious web (117) as claimed in claim 1, wherein said fibers (124) are meltblown fibers.

4. A fluid pervious web (112) as claimed in any of claims 1-3, wherein said plastic web (112) is comprised of a material selected from a group consisting of polyethylene, polypropylene, polyvinylchloride, starch base resins, polyvinylalcohol, polyurethanes, polycaprolactone and cellulose esters.

5. An absorbent article (10) which comprises a topsheet (12), a fluid impervious backsheet (14) joined to said topsheet (12), and an absorbent core (16) positioned between the top sheet (12) and the backsheet (14), said absorbent article **characterised in that** the topsheet (12) comprises the web (112) as claimed in any of claims 1-4.

6. An article as claimed in Claim 5, wherein said absorbent article (10) is a disposable diaper.

7. An article as claimed in Claim 5, wherein said absorbent article (10) is a sanitary napkin.

8. A process for forming a fibrous and apertured, three-dimensional, macroscopically expanded web (112), said process comprising the steps of:
(a) extruding a polymeric film (200) onto a forming structure (210), said forming structure (210) exhibiting a macroscopic, three-dimensional, cross-section defined by a multiplicity of macroscopic apertures which place the opposed surfaces of said forming structure in fluid communication with one another;
(b) depositing a plurality of fibers (202) on said film to form a composite web (206); and
(c) applying a pneumatic pressure to said composite web (206) to cause said composite web (206) to be urged into substantial conformance with the macroscopic, three-dimensional, cross-section of said forming structure forming a three-dimensional, macroscopically expanded, formed web (112) having first (140) and second (145) surfaces, said formed web (112) having a plurality of capillaries (149) extending from said first surface (140) to said second surface (145), said capillaries (149) being defined by a plurality of sidewall portions (143) interconnected to one another intermediate said first (140) and second (145) surfaces and terminating in said second surface (145), said pneumatic pressure causing said fibers (202) to be pulled against said first surface (140) of said formed web (206) and into said capillaries (149) of said formed web (206), said fibers (202) being secured to said first surface (140) and a substantial portion of said fibers (202) being secured to said sidewall portions (143) of said formed web (206) forming said fibrous and apertured, three-dimensional, macroscopically expanded web (112).

9. A process for forming a fibrous and apertured, three-dimensional, macroscopically expanded web (112), said process **characterized by** the steps of
(a) meltblowing a plurality of fibers onto a forming drum;
(b) extruding a polymeric film (200) onto said plurality of fibers to form a composite web (206);
(c) placing said composite web (206) on a forming structure, said forming structure exhibiting a macroscopic, three-dimensional, cross-section defined by a multiplicity of macroscopic apertures which place the opposed surfaces of said forming structure in fluid communication with one another; and
(d) applying a pneumatic pressure to said composite web (206) to cause said composite web (206) to be urged into substantial conformance with the macroscopic, three-dimensional, cross-section of said forming structure forming a three-dimensional, macroscopically expanded, formed web having first (140) and second (145) surfaces, said formed web having a plurality of capillaries (149) extending from said first surface (140) to said second surface (149), said capillaries (149) being defined by a plurality of sidewall portions (143) interconnected to one another intermediate said first (140) and second (145) surfaces and terminating in said second surface (145).

## Patentansprüche

1. Elastische, dreidimensionale, makroskopisch ausgedehnte, fluiddurchlässige Kunststoffbahn (112) mit einer ersten (140) und einer zweiten (145) Oberfläche, welche in Ebenen entfernt voneinander angeordnet sind, wobei die Bahn (112) eine Vielzahl von Kapillaren (149) aufweist, die sich von der ersten Oberfläche (140) zur zweiten Oberfläche (145) erstrecken, wobei die Kapillaren (149) durch eine Vielzahl von Seitenwandabschnitten (143) bestimmt werden, die zwischen der ersten (140) und der zweiten (145) Oberfläche miteinander verbunden sind, wobei die Seitenwandabschnitte (143) in der zweiten Oberfläche (145) enden;
eine Vielzahl von Fasern (124), die an der ersten Oberfläche (140) der Bahn (112) befestigt sind, **dadurch gekennzeichnet, dass** die Fasern (124) sich in die Kapillaren (149) hinein erstrecken und dass ein wesentlicher Teil der Fasern (124) an den Seitenwandabschnitten (143) befestigt ist.

2. Fluiddurchlässige Bahn (112), wie in Anspruch 1 beansprucht, wobei die Fasern (124) in einem Vliesstoff vorliegen.

3. Fluiddurchlässige Bahn (112), wie in Anspruch 1 beansprucht, wobei die Fasern (124) schmelzgeblasene Fasern sind.

4. Fluiddurchlässige Bahn (112), wie in einem der Ansprüche 1-3 beansprucht, wobei die Kunststoffbahn (112) aus einem Material besteht, das ausgewählt ist aus der Gruppe bestehend aus Polyethylen, Polypropylen, Polyvinylchlorid, stärkebasierten Harzen, Polyvinylalkohol, Polyurethanen, Polycaprolacton und Celluloseestern.

5. Absorptionsartikel (10), welcher eine Oberschicht (12), eine fluidundurchlässige Unterschicht (14), die mit der Oberschicht (12) verbunden ist, und einen zwischen der Oberschicht (12) und der Unterschicht (14) positionierten Absorptionskem (16) umfasst, wobei der Absorptionsartikel **dadurch gekennzeichnet ist, dass** die Oberschicht (12) die Bahn (112) umfasst, wie in einem der Ansprüche 1-4 beansprucht.

6. Artikel, wie in Anspruch 5 beansprucht, wobei der Absorptionsartikel (10) eine Einwegwindel ist.

7. Artikel, wie in Anspruch 5 beansprucht, wobei der Absorptionsartikel (10) eine Damenbinde ist.

8. Verfahren zur Bildung einer faserigen und durchlässigen, dreidimensionalen, makroskopisch ausgedehnten Bahn (112), wobei das Verfahren die folgenden Schritte umfasst:
(a) Extrudieren eines Polymerfilms (200) auf eine Formstruktur (210), wobei die Formstruktur (210) einen makroskopischen, dreidimensionalen Querschnitt aufweist, der durch eine Vielzahl von makroskopischen Öffnungen bestimmt wird, die einen Fluidaustausch zwischen den gegenüberliegenden Oberflächen der Formstruktur bewirken;
(b) Anlagern einer Vielzahl von Fasern (202) auf dem Film, um eine Verbundstoffbahn (206) zu bilden; und
(c) Anwenden eines pneumatischen Drucks auf die Verbundstoffbahn (206), um zu bewirken, dass die Verbundstoffbahn (206) in wesentliche Übereinstimmung mit dem makroskopischen, dreidimensionalen Querschnitt der Formstruktur gebracht wird, wodurch eine dreidimensionale, makroskopisch ausgedehnte, geformte Bahn (112) mit einer ersten (140) und einer zweiten (145) Oberfläche gebildet wird, wobei die geformte Bahn (112) eine Vielzahl von Kapillaren (149) aufweist, die sich von der ersten Oberfläche (140) zur zweiten Oberfläche (145) erstrecken, wobei die Kapillaren (149) durch eine Vielzahl von Seitenwandabschnitten (143) bestimmt werden, die zwischen der ersten (140) und der zweiten (145) Oberfläche miteinander verbunden sind und in der zweiten Oberfläche (145) enden, wobei der pneumatische Druck bewirkt, dass die Fasern (202) gegen die erste Oberfläche (140) der geformten Bahn (206) und in die Kapillaren (149) der geformten Bahn (206) gezogen werden, wobei die Fasern (202) an der ersten Oberfläche (140) befestigt sind und ein wesentlicher Teil der Fasern (202) an den Seitenwandabschnitten (143) der geformten Bahn (206) befestigt ist, wodurch die faserige und durchlässige, dreidimensionale, makroskopisch ausgedehnte Bahn (112) gebildet wird.

9. Verfahren zur Bildung einer faserigen und durchlässigen, dreidimensionalen, makroskopisch ausgedehnten Bahn (112), wobei das Verfahren **gekennzeichnet ist durch** die Schritte
(a) Schmelzblasen einer Vielzahl von Fasern auf eine Formtrommel;
(b) Extrudieren eines Polymerfilms (200) auf die Vielzahl von Fasern, um eine Verbundstoffbahn (206) zu bilden;
(c) Platzieren der Verbundstoffbahn (206) auf einer Formstruktur, wobei die Formstruktur einen makroskopischen, dreidimensionalen Querschnitt aufweist, der **durch** eine Vielzahl von makroskopischen Öffnungen bestimmt wird, die einen Fluidaustausch zwischen den gegenüberliegenden Oberflächen der Formstruktur bewirken; und
(d) Anwenden eines pneumatischen Drucks auf die Verbundstoffbahn (206), um zu bewirken, dass die Verbundstoffbahn (206) in wesentliche Übereinstimmung mit dem makroskopischen, dreidimensionalen Querschnitt der Formstruktur gebracht wird, wodurch eine dreidimensionale, makroskopisch ausgedehnte, geformte Bahn mit einer ersten (140) und einer zweiten (145) Oberfläche gebildet wird, wobei die geformte Bahn eine Vielzahl von Kapillaren (149) aufweist, die sich von der ersten Oberfläche (140) zur zweiten Oberfläche (145) erstrecken, wobei die Kapillaren (149) **durch** eine Vielzahl von Seitenwandabschnitten (143) bestimmt werden, die zwischen der ersten (140) und der zweiten (145) Oberfläche miteinander verbunden sind und in der zweiten Oberfläche (145) enden.

## Revendications

1. Bande plastique (112) perméable aux fluides, à expansion macroscopique, tridimensionnelle, élastique ayant une première (140) et une deuxième (145) surfaces situées dans des plans distants l'un de l'autre, ladite bande (112) ayant une pluralité de capillaires (149) s'étendant de ladite première surface (140) à ladite deuxième surface (145), lesdits capillaires (149) étant définis par une pluralité de parties de paroi latérale (143) interconnectées les unes aux autres de façon intermédiaire entre lesdites première (140) et deuxième (145) surfaces, lesdites parties de paroi latérale (143) se terminant dans ladite deuxième surface (145) ;
une pluralité de fibres (124) attachées à ladite première surface (140) de ladite bande (112), **caractérisée en ce que** lesdites fibres (124) s'étendent dans les capillaires (145) et une partie essentielle desdites fibres (124) sont attachées auxdites parties de paroi latérale (143).

2. Bande perméable aux fluides (112) selon la revendication 1, dans laquelle lesdites fibres (124) sont en étoffe nontissée.

3. Bande perméable aux fluides (117) selon la revendication 1, dans laquelle lesdites fibres (124) sont des fibres soufflées en fusion.

4. Bande perméable aux fluides (112) selon l'une quelconque des revendications 1 à 3, où ladite bande plastique (112) est constituée d'un matériau choisi parmi un groupe constitué de polyéthylène, polypropylène, chlorure de polyvinyle, résines à base d'amidon, alcool polyvinylique, polyuréthanes, polycaprolactone et esters de cellulose.

5. Article absorbant (10) qui comprend une feuille de dessus (12), une feuille de fond imperméable aux fluides (14) jointe à ladite feuille de dessus (12), et une âme absorbante (16) positionnée entre la feuille de dessus (12) et la feuille de fond (14), ledit article absorbant **caractérisé en ce que** la feuille de dessus (12) comprend la bande (112) selon l'une quelconque des revendications 1 à 4.

6. Article selon la revendication 5, dans lequel ledit article absorbant (10) est une couche jetable.

7. Article selon la revendication 5, où ledit article absorbant (10) est une serviette hygiénique.

8. Procédé de formage d'une bande (112) à expansion macroscopique, tridimensionnelle, fibreuse et perforée, ledit procédé comprenant les étapes consistant à
(a) extruder un film polymère (200) sur une structure de formage (210), ladite structure de formage (210) présentant une coupe transversale tridimensionnelle, macroscopique, définie par une multiplicité d'orifices macroscopiques qui placent les surfaces opposées de ladite structure de formage en communication du point de vue des fluides l'une avec l'autre ;
(b) déposer une pluralité de fibres (202) sur ladite pellicule pour former une étoffe composite (206) ; et
(c) appliquer une pression pneumatique à ladite étoffe composite (206) pour faire en sorte que ladite étoffe composite (206) soit activée dans une mise en conformité substantielle avec la coupe transversale tridimensionnelle macroscopique de ladite structure de formage formant une bande (112) formée à expansion macroscopique, tridimensionnelle ayant une première (140) et une deuxième (145) surfaces, ladite bande formée (112) ayant une pluralité de capillaires (149) s'étendant de ladite première surface (140) à ladite deuxième surface (145), lesdits capillaires (149) étant définis par une pluralité de parties de paroi latérale (143) interconnectées les unes aux autres de façon intermédiaire entre lesdites première (140) et deuxième (145) surfaces et se terminant dans ladite deuxième surface (145), ladite pression pneumatique provoquant le poussage desdites fibres (202) contre ladite première surface (140) de ladite bande formée (206) et dans lesdits capillaires (149) de ladite bande formée (206), lesdites fibres (202) étant attachées à ladite première surface (140) et une partie substantielle desdites fibres (202) étant attachées auxdites parties de paroi latérale (143) de ladite bande formée (206) formant ladite bande à expansion macroscopique, tridimensionnelle, fibreuse et perforée (112).

9. Procédé de formage d'une bande (112) à expansion macroscopique tridimensionnelle, fibreuse et perforée, ledit procédé **caractérisé par** les étapes consistant à :
(a) souffler en fusion une pluralité de fibres sur un tambour de formage ;
(b) extruder un film polymère (200) sur ladite pluralité de fibres pour former une étoffe composite (206) ;
(c) placer ladite étoffe composite (206) sur une structure de formage, ladite structure de formage présentant une coupe transversale tridimensionnelle macroscopique, définie par une multiplicité d'orifices macroscopiques qui placent les surfaces opposées de ladite structure de formage en communication l'une avec l'autre du point de vue des fluides ; et
(d) appliquer une pression pneumatique à ladite étoffe composite (206) pour faire en sorte que ladite étoffe composite (206) soit forcée dans une mise en conformité substantielle avec la coupe transversale tridimensionnelle macroscopique de ladite structure de formage formant une bande formée à expansion macroscopique tridimensionnelle, ayant une première (140) et une deuxième (145) surfaces, ladite bande formée ayant une pluralité de capillaires (149) s'étendant de ladite première surface (140) à ladite deuxième surface (149), lesdits capillaires (149) étant définis par une pluralité de parties de parois latérales (143) interconnectées les unes aux autres de façon intermédiaire entre lesdites première (140) et deuxième (145) surfaces et se terminant dans ladite deuxième surface (145).
